# EUROPEAN PATENT SPECIFICATION

(11) **EP 3 960 209 B1**
(45) Date of publication and mention of the grant of the patent: **19.07.2023**
(21) Application number: 21190499.0
(22) Date of filing: 10.08.2021
(51) Int. Cl.: A61L 2/10, A61L 2/24, A61L 9/20, H05B 47/19, H05B 47/195, H05B 47/115, H05B 47/20

(54) **A SYSTEM FOR DETERMINING A CONDITION OF A SENSOR OF A LIGHTING DEVICE**
SYSTEM ZUR BESTIMMUNG EINES ZUSTANDS EINES SENSORS EINER BELEUCHTUNGSVORRICHTUNG
SYSTÈME PERMETTANT DE DÉTERMINER L'ÉTAT D'UN CAPTEUR D'UN DISPOSITIF D'ÉCLAIRAGE

(30) Priority: 24.08.2020 EP 20192305
(43) Date of publication of application: 02.03.2022
(73) Proprietor: Signify Holding B.V., 5656 AE Eindhoven (NL)
(72) Inventor: TENHUMBERG, Christian, 5656 AE Eindhoven (NL)
(74) Representative: Özcan, Evren

(56) References cited:
- WO-A1-2017/076715
- WO-A1-2021/195003
- US-A1- 2018 296 711
- US-B2- 10 448 006

## Description

### FIELD OF THE INVENTION

The invention relates to a system. The system is particularly arranged for determining a condition of a sensor of a lighting device. The invention further relates to a system arrangement comprising a plurality of systems according to said system.

The invention further relates to a corresponding method of determining a condition of a sensor of a lighting device, and to a computer program product.

### BACKGROUND OF THE INVENTION

The COVID-19 pandemic has been shaking the world in 2020. However, health and wellbeing of people has been contested periodically with other viruses and bacteria outbreaks as well, such as for example the seasonal symptomatic influenza A/B outbreak, SARS, H1N1 and MERS. Similar outbreaks are also found in the animal domain, such as e.g. outbreaks in Avian Influenza (i.e. Bird Flu) and Coxiella Burnetii (i.e. Q-fever). Future outbreaks, epidemics, and pandemics are not excluded.

The COVID-19 pandemic has already shown that it may cause economic recession; while seasonal symptomatic Influenza has proven to cause a recurring economic burden; while outbreaks of contagious animal diseases have occasionally disrupted local animal farming; while even further new diseases loom inevitably on the horizon.

Therefore, to prevent economic loss, and to improve health of people and animals, a clear need exists for devices promoting health and wellbeing in frequently used spaces. An ultraviolet (UV) disinfection device may be such a device, particularly when using UV-C. Namely, ultraviolet disinfection devices are increasingly and successfully being used to disinfect surfaces and the air in spaces. Applications may be found in offices, hospitals, retail, home and public spaces.

However, since various spectra of UV illumination for disinfection may also be hazardous to humans and animals, in certain doses and times of exposure, such UV disinfection devices may require stringent safety measures.

A safety measure, which is commonly applied, is an indicator light on the disinfection device that indicates that the device is currently operational. Another safety measure is to make use of a presence sensor, to ensure that a disinfection device is turned off when human presence is detected in a space that is being disinfected.

However, such sensors may be prone to a malfunction during the lifetime of the ultraviolet disinfection device, e.g. because their electronic components are subject to ageing and prone to become outdated. Hence, the correct working of such sensors needs to be checked regularly.

Moreover, the properties of the space such sensors are monitoring may frequently change, such as e.g. in offices, hospitals, and retail. Such changes may affect the field-of-view of said sensors. For example, such changes may block a part of the field-of-view of such sensors. Even though a human may be present in the space, the sensor would then not be able to detect the person, and render a risk of the human being exposed to undesired doses of ultraviolet light. Therefore, a clear need exists to verify the correct functioning of said sensors, and also their correct functioning within the full scope of their field-of-view.

US2019045180 (A1) discloses a method of testing a sensor unit which detects, locally at the sensor unit, whether a person appears in the images it captures. The method comprises causing a light-emitting device (e.g. screen of a user terminal) to emit a test pattern toward the sensor unit to be captured over a series of images, the test pattern comprising a spatio-temporal pattern of light simulating presence of a predetermined number of people.

WO2017076715 (A1) discloses a similar people sensing system making use of test images.

Finally US2018296711 discloses a room decontamination apparatus with a safety redundancy based on multiple sensors to check failure or malfunctioning of the presence sensor.

### SUMMARY OF THE INVENTION

It is an object of the invention to provide a system, which at least alleviates the problems and disadvantages mentioned above. Thereto, the invention provides a system comprising: a lighting device comprising a sensor and a light source, wherein the sensor is configured to detect a presence in a space and to detect an emulator signal, wherein the light source is configured to provide ultraviolet light in operation and to interrupt providing said ultraviolet light in operation when the sensor detects presence in said space; an emulator device configured to convey the emulator signal to the sensor; a controller configured to control the emulator device to transmit the emulator signal at a first moment in time, and to determine a verification condition of the sensor if the sensor detects the emulator signal at the first moment in time.

Hence, the lighting device comprising the sensor and the light source enables ultraviolet disinfection and/or ionization due to the light source being configured to provide ultraviolet light in operation, while the lighting device enables safety due to the light source being configured to interrupt providing said ultraviolet light in operation when the sensor detects presence in the space. Said sensor may alternatively be phrased as a presence sensor or occupancy sensor. Said in operation may mean during a disinfection action and/or during an ionization action.

However, the system according to the invention provides an emulator device and a controller, which controller controls the emulator device to transmit an emulator signal at a first moment in time to the sensor. The emulator signal is thus purposively transmitted to the sensor at the first moment in time. The emulator device may thus emulate the presence in the space. The emulator signal is detectable by the sensor. This transmission may advantageously be used to determine a condition of the sensor of the lighting device. Namely, the controller is configured to determine a verification condition of the sensor if the sensor detects the emulator signal at that first moment in time. The sensor is thereby tested accordingly. Therefore, the system according to the invention is able to verify the correct working or functioning of the sensor (by determining said verification condition).

The verification condition is indicative of the sensor being functioning properly (in correctly detecting the presence in the space). However, the sensor may also malfunction. Such a condition may also be determined. Hence, in an embodiment, the controller may be configured to determine an error condition of the sensor if the sensor does not detect the emulator signal at the first moment in time.

The components of the system may also advantageously respond to the determination of such an error condition. Hence, in an embodiment, the controller may be configured to output an output signal upon determining said error condition. Therefore, the system may determine an error condition of the sensor, which may indicate incorrect functioning of the sensor (in detecting the presence), and act thereupon by outputting the output signal.

Determining said verification condition or said error condition may be phrased as a verification the correct functioning of the sensor. The space wherein the sensor detects the presence may be part of, or comprised by, a main space. The system may thus be applied in a main space. The space may be phrased as detection region. Said first moment in time may alternatively be a plurality of first moments in time.

Said ultraviolet light may be light with a spectral distribution comprising an ultraviolet spectrum. Said light may further comprise a further lighting characteristic being at least one of: an intensity, a modulation, a color temperature, a pattern, a recipe, a beam shape, a scene, etc.

Said emulator device may be battery powered. Said emulator device may comprise means for detachably mounting said emulator device to a surface. Said emulator device may be part of a light switch, wherein said light switch is also comprised by the system according to the invention.

In aspects, the emulator device may be a portable device, or be part of a portable device. In aspects, the controller may determine a location of said portable device within the field-of-view of the sensor, and then control the emulator device to transmit the emulator signal at a first moment in time.

In an embodiment, said output signal may be arranged for controlling the lighting device to disable the light source to provide said ultraviolet light in operation. Therefore, upon determining that the sensor may not be functioning accordingly (in detecting the presence), the light source may be disabled to provide said ultraviolet light in operation. This advantageously prevents a risk of operating the light source to provide ultraviolet light while presence in the space may not be detected accordingly. The output signal may thus be a control signal.

Additionally, or alternatively, in an embodiment, said output signal may be indicative of the error condition, wherein the controller is configured to output the output signal to a further device so as to notify the further device on the error condition. Therefore, upon determining that the sensor may not be functioning accordingly (in detecting the presence), this condition itself may be outputted (or: conveyed) to a further device, so as to notify (or: inform) said further device about a possible risk emanating from an incorrect functioning sensor of the lighting device according to the invention. The output signal may thus be a notification signal.

Said further device may for example be at least one of: a portable device, a user device, a computer, a smartphone, a wearable device, a smartwatch, smart glasses, a tablet, a building management device, a maintenance server, a smart lock, a smart door, a smart window, a lighting device, a switch device, a server, a memory, a room booking device, a vehicle, a drone.

In aspects: The controller may be configured to control the lighting device to disable the light source to provide (or: in providing) said ultraviolet light in operation upon determining said error condition. In aspects: The controller may be configured to disable the lighting device upon determining said error condition. In aspects: The controller may be configured to temporarily disable the lighting device upon determining said error condition. In aspects: The controller may be configured to disable or temporarily disable a mode of the lighting device upon determining said error condition. Said mode may e.g. be a UV disinfection mode. In aspects: The controller may be configured to enable an auxiliary sensor upon determining said error condition. In aspects, said temporarily may mean one hour, at least one hour, one day, one week, at least one day, or at least two hours.

Said ultraviolet light may for example comprise UV-A (315-400 nm), UV-B (280-315 nm), UV-C (100-280 nm), Far-UV, Extreme-UV. More specifically, in aspects, said spectral distribution may comprise at least one of: Ultra Deep UV in the range of 100 - 190 nm, Deep UV light in the range of 190-220 nm, UV-C light in the range of 220-280 nm, UV-B light in the range of 280-315 nm, UV-A light in the range of 315-400 nm, UV light at 254 nm. Such an ultraviolet spectrum of light may have proven effectively in killing infectious matter, such as viruses, bacteria, fungi, yeasts and/or germs. For example, UV-C lighting comprises a strong bactericidal effect, damaging the structure of DNA in living cells.

Therefore, the ultraviolet light may also be harmful for humans and animals. Hence, the present invention provides a lighting device, and a system comprising such a lighting device, that operates with such risky ultraviolet light and comprises a sensor for safety. Particularly this sensor needs to be checked regularly on whether it still functions correctly.

Said ultraviolet light may alternatively be deep blue light in the range 400-420 nm, deep blue light at 405 nm. This light spectrum may also be undesired for a human and/or an animal at particular doses and times of exposure.

As mentioned, said first moment in time may alternatively be a plurality of first moments in time. Hence, the verification of the correct functioning of the sensor may be performed continually in time, e.g. periodically or according to a schedule. For example, the controller may determine said first moment in time based on a schedule, wherein said schedule may be a schedule defining when the light source is in operation. For example, said first moment in time may be before a period of time in which the light source is in operation. This ensures safety, as the verification is performed before the light source of the lighting device is operated, i.e. the lighting device performs e.g. a disinfection action.

In an embodiment, the sensor may be a PIR sensor, the emulator device may be an infrared light source, and the emulator signal may be an infrared light signal. Since PIR sensors may commonly be used to detect a presence in a space by observing motion of people and/or animals, such a sensor may be suitable for the lighting device according to the invention. The corresponding emulator device may therefore be an infrared light source, which may be effective in operation, compact and cost-effective.

Radiofrequency based sensors are increasingly applied in the office and/or agricultural domain. In an embodiment, the sensor may be microwave sensor, the emulator device may be a radiofrequency beacon, and the emulator signal may be a radiofrequency signal. Since microwave sensors may effectively be used to detect a presence in a space by observing motion of people and/or animals, such a sensor may be suitable for the lighting device according to the invention. The corresponding emulator device may therefore be a radiofrequency beacon, which may be effective in operation, compact and widely available.

Because radiofrequency beacons may already be available in a space, for example as being part of an asset tracking system, such radiofrequency beacons may also serve as the emulator device according to the invention.

In an embodiment, the sensor may be a light sensor, and the emulator device may be a light beacon, wherein the emulator signal may be a light signal. Since light sensors may commonly and cost-effectively be used to detect a presence in a space by observing changes in illumination, such a sensor may be suitable for the lighting device according to the invention. The corresponding emulator device may therefore be a light beacon. The sensor may alternatively be a camera.

Said light beacon may be a lamp or a luminaire. Hence, the controller may control a lamp or a luminaire to transmit a light beacon at the first moment in time, and determine a verification condition of the sensor if the sensor detects the light beacon at the first moment in time, and determine an error condition of the sensor if the sensor does not detect the light beacon at the first moment in time. Said lamp or luminaire may already have a first function of illuminating said space, or illuminating at least part of a main space comprising the space, wherein providing the emulator signal as the light beacon may serve as a second function, which second function facilitates the verification of the sensor the lighting device comprising the light source providing the ultraviolet light in operation.

In aspects, the sensor may be thermopile sensor, the emulator device may be a heat beacon, and the emulator signal may be a heat signal. The heat beacon may comprise a heating element. Said heating element may heat up. Said heating element may provide heat radiation. The heat beacon may be a laser. The heat beacon may also project a heat signal onto a surface, e.g. by projecting radiative heat onto a surface.

In aspects, the sensor may be a microphone, the emulator device may be a sound source, and the emulator signal may be a sound signal.

In aspects, the sensor may be a Time-of-Flight sensor, the emulator device may be a light source, and the emulator signal may be a light signal. The light signal of the light source may be detectable by the time-of-flight sensor. For example, the light signal of the light source may be tailored to a light signal transmitted by the time-of-flight sensor, and mimic said light signal transmitted by the time-of-flight sensor, so as to emulate that light signal transmitted by the time-of-flight sensor.

The lighting device according to the invention may be suited for ultraviolet disinfection. Ultraviolet disinfection may comprise disinfection of a surface and/or a fluid in the space or a main space comprising the space. Said fluid may for example be the air in the space, and/or water in the space. Said fluid may for example be the air in the main space comprising the space, and/or water in the main space comprising the space. Hence, in an embodiment, the light source may be configured to illuminate in operation at least part of the space with the ultraviolet light; or to illuminate at least part of a main space comprising the space with the ultraviolet light. The light source may thereby directly disinfect at least part of the main space and/or the space. This promotes health and wellbeing, but may render a higher risk of exposure to ultraviolet light. However, the risks of the ultraviolet disinfection are increasingly mitigated by the system according to the invention, which determines either a verification condition or an error condition of the (presence) sensor that is ought to provide safety.

The lighting device of the system according to the invention may be a UV disinfection luminaire. In an embodiment, the lighting device may be a suspended luminaire. Such a lighting device may preferably illuminate towards a ceiling of a main space, the main space comprising the space (in which the sensor detects the presence). Hence, in an embodiment, the light source may be configured to provide the ultraviolet light in a direction opposite to gravity.

In aspects: the light source may comprise an optic to provide the ultraviolet in a direction opposite to gravity. Said optic may e.g. constitute of lenses, reflectors, lighting rods, and/or light guides. In aspects: the lighting device may comprise an optic to direct the ultraviolet light of the light source in a direction opposite to gravity.

Ultraviolet light may also be successfully be implemented to purify air. This may be done by receiving air from a main space, purifying said air by e.g. direct exposure to disinfecting UV light and/or creating ozone via UV activation, and outputting said air back into said main space. The main space comprising the space according to the invention. This concept may be applied in an air purifier (i.e.: referred to as purifier for convenience). Said purifier may alternatively be a water purifier. Therefore, additionally or alternatively, the lighting device according to the invention may be suited for accommodating a purifier purifying (or: cleaning) the air of (at least part of) the main space with the use of the ultraviolet light of the light source according to the invention. Even though the ultraviolet light may not always directly visible from the main space, because the ultraviolet light may be provided within the purifier, such devices may in operation still pose a safety risk to humans and animals. The system according to the invention may thus also be advantageous for such configurations. Hence, in an embodiment, the lighting device may comprise a purifier for purifying air in operation, wherein the purifier comprises the light source, wherein the ultraviolet light of the light source may purify the air.

In aspects: the lighting device may comprise an ozone generator for in operation creating ozone from air, wherein the ozone generator comprises the light source, wherein the ultraviolet light of the light source may create ozone from the air.

The system according to the invention comprises: the lighting device comprising the sensor and the light source; the emulator device; and the controller. These components may be operative coupled and may be in interaction with each other. The controller may be arranged separately from the lighting device and/or the emulator device. The controller may communicate with the lighting device and/or the emulator device via a wired connection. Alternatively, the controller may communicate with the lighting device (and the sensor) and/or the emulator device via or may communicate via a wireless connection, such as e.g. Bluetooth, ZigBee, Wi-Fi, RF, IR, Lo-Ra, UWB, 5G, Li-Fi, VLC, etc.

In an embodiment, the lighting device may comprise the controller. Hence, the controller may be a local controller of the lighting device. Such a configuration may prevent communication being required with a remotely arranged controller. Phrased differently: the lighting device may comprise a housing, wherein the housing comprises the controller. In such differently phrased examples, the housing may similarly comprise the sensor and the light source according to the invention.

In an embodiment, the space may comprise a surface, wherein the emulator device may be configured to project said emulator signal onto the surface. As mentioned before, the system may comprise a main space, which main space may comprise the space. The main space may comprise the surface. The emulator device may thereby be configured to project said emulator signal onto a projection location on said surface. The projection may thereby be at the first moment of time, as indicated before. Such an embodiment may be advantageous, because the emulator device may convey the emulator signal to the sensor without being on the projected location on the surface itself, since the emulator device is projecting the emulator signal onto that location on the surface from another location. This provides flexibility in positioning of the emulator device.

The surface may reflect said emulator signal. The surface may visualize said emulator signal. The emulator signal may thereby be, for example, a light signal or an infrared signal, which is projected onto a surface. The surface may be a wall, a ceiling, a floor, a door, a desk, a widow, a curtain, furniture, etc. In examples of the present embodiment, said emulator device may for example be a laser, and said emulator signal may be a laser signal projected onto (a projection location on) said surface. The sensor may thereby be a PIR sensor, a camera, a thermopile sensor, or a light sensor.

The space may be being monitored by the sensor according to the invention. The space may be part of a main space. The sensor may for example comprise a detection region in which the sensor is configured to detect the presence in the space and/or the emulator signal. The sensor may thus monitor its field-of-view. Hence, the space may be considered the detection region or the field-of-view.

As partly mentioned, properties of a space may frequently change, such as e.g. in offices, hospitals and retail. This may also apply to the main space according to the invention. The main space comprises the space. Such changes may affect the field-of-view of the sensor, or affect the detection region of the sensor. For example, a part of the field-of-view or detection region may be blocked. Hence, even though a human may be present in the space, the sensor would not be able to correctly detect the person in such a situation, and render a risk of the human being exposed to undesired doses of ultraviolet light. This is a clear problem. It may therefore be advantageous to verify the correct functioning of the sensor, which verification is valid for larger parts of the field-of-view or the detection region.

Hence, in an embodiment, the emulator device may be arranged at a distance from the sensor, wherein said distance is at least two meters. Therefore, the verification of the correct functioning of the sensor is performed over the full distance between the emulator device and the sensor. A larger distance may thereby be more representative for the space. Alternatively, said distance may be at least four meters. Alternatively, said distance may be at least eight meters or at least ten meters. In aspects, said distance is at least ten times a largest length of the lighting device.

Therefore, the system according to the present invention provides a more robust safety check for the sensor. For example, if the distance between the emulator device and the sensor is two meters, the mentioned verification may consider (or: take into account) any possible blocking objects in that particular two meters from the sensor.

Alternatively, the distance between the emulator device and the sensor may mutatis mutandis be described in terms of the propagated distance of the emulator signal. The emulator signal may thus comprise a propagation distance. This may be even more representative. The advantages apply similarly.

Hence, in examples, the emulator signal may comprise a propagation distance between the emulator device and the sensor, wherein the emulator device is arranged from the sensor such that the propagation distance is at least two meters. Alternatively, said propagation distance may be at least four meters. Alternatively, said distance may be at least eight meters or at least ten meters.

Hence, in examples, a propagation distance that the emulator signal propagates through the space between the emulator device and the sensor may be at least two meters. Alternatively, said propagation distance may be at least four meters. Alternatively, said distance may be at least eight meters or at least ten meters. In aspects, a propagation distance that the emulator signal propagates through a main space between the emulator device and the sensor may be at least two meters, the main space comprising the space.

Moreover, in an embodiment, the space or a main space comprising the space may be delineated by a delineating surface, wherein the emulator device may be arranged on the delineating surface. Since the delineating surface bounds the space or said main space, a maximum extent of these spaces may be considered in the verification of the sensor according to the invention. In an embodiment, a main space comprising the space comprises a surface, wherein the emulator device is mounted to said surface. The delineating surface or said surface may be a wall, a ceiling, a floor, a door, a desk, a widow, a curtain, furniture, etc.

In an embodiment, the emulator device may be remote from the lighting device. In an embodiment, the emulator device may be part of a further apparatus. In an embodiment, the system may comprise a further apparatus, wherein the further apparatus comprises the emulator device.

In an embodiment, the system comprises a second lighting device; wherein the second lighting device comprises the emulator device. Hence, the invention may provide a set of a first lighting device and a second lighting device. Such a set may render that the second lighting device facilitates the verification of the correct functioning of the sensor of the first lighting device, because the second lighting device comprises the emulator device. Since lighting devices are often mounted at the same height in spaces, it may be advantageous to have a second lighting device comprising the emulator device.

In an embodiment, the lighting device may comprise the emulator device. Such an embodiment may be advantageous, because the sensor and the emulator device may be comprised by (and accommodated by) the same lighting device. The lighting device itself is thereby autonomously able to perform self-tests of the sensor it comprises.

Moreover, in such an embodiment, the emulator device may project said emulator signal onto a surface. The emulator signal may therefore be conveyed to the sensor via said projection onto said surface. The emulator signal may for example be a laser pattern, which is projected onto a part of a surface in the space, such that the sensor may detect the projected laser pattern, and wherein the controller may determine said verification condition upon the sensor detecting the projected laser pattern at the first moment in time.

In an embodiment, the lighting device may be a luminaire and comprises a luminaire housing, wherein the luminaire housing comprises the sensor, the light source, the controller and the emulator device.

In the examples wherein the system comprises a second lighting device comprising the emulator device of the lighting device, the lighting device may also comprise an emulator device, but yet from another lighting device. The system according to the invention may thus render a chain of lighting devices with emulator devices and sensors, which lighting devices facilitate the verification of the correct functioning of the sensors of adjacent lighting devices by comprising the emulator devices of said adjacent lighting devices. Hence, a repetitive and modular configuration may be achieved, which may be advantageously applied within the context of lighting, because lighting in indoor spaces often comprises spatially organized groups of lighting devices.

Therefore, it is a further object of the invention to provide a system arrangement, which at least alleviates the problems and disadvantages mentioned above. Thereto, the invention further provides a system arrangement comprising a plurality of systems according to the invention, wherein the respective lighting device of at least one system of the plurality of systems comprises the respective emulator device of one other system of the plurality of systems. The advantages and/or embodiments applying to the system according to the invention may also apply mutatis mutandis to the system arrangement according to the invention.

In an embodiment, the plurality of systems may be spatially arranged in a main space according to a grid pattern. Hence, the lighting devices may be organized in a grid pattern. The grid pattern may be defined in a N x M matrix, wherein N and M are integers. For example, the grid pattern may be one of a 2x2 matrix, a 4x4 matrix, a 1x2 matrix, a 1x4 matrix. Other examples may be envisioned similarly with combination of the integers N and M.

In an embodiment, the system arrangement comprises a main controller, wherein the main controller comprises each respective controller of the plurality of systems. Such a centralized system may be advantageous.

Each system of the plurality of systems may comprise a controller. However, said controller may be a local controller (or: local microcircuit) receiving commands from a central controller. The central controller and the local controllers may thereby be considered as one main controller. The local controllers may be part of the central controller, or may have a part of their controller (circuit) in the central controller.

In aspects: the invention provides a system arrangement comprising a plurality of systems, wherein each system of the plurality of systems comprises a lighting device comprising a sensor and a light source, wherein the sensor is configured to detect a presence in a space and/or to detect an emulator signal, wherein the light source is configured to provide ultraviolet light in operation and to interrupt providing said ultraviolet light in operation when the sensor detects presence in said space; an emulator device configured to convey the emulator signal to the sensor; wherein the respective lighting device of at least one system of the plurality of systems comprises the respective emulator device of one other system of the plurality of systems; wherein the system arrangement comprises a controller configured to control at least one respective emulator device of a system of the plurality of systems to transmit the respective emulator signal at a first moment in time, and to determine a verification condition of the respective sensor of the lighting device of said system of the plurality of systems if said sensor detects the respective emulator signal at the first moment in time and to determine an error condition of the respective sensor of the lighting device of said system of the plurality of systems if said sensor does not detect the emulator signal at the first moment in time. The advantages and/or embodiments applying to the system according to the invention may also apply mutatis mutandis to the system arrangement according to the invention.

It is a further object of the disclosure to provide a lighting device, which at least alleviates the problems and disadvantages mentioned above. Thereto, an aspect further provides a lighting device comprising a sensor, a light source, a controller and an emulator device; wherein the sensor is configured to detect a presence in a space and/or to detect an emulator signal, wherein the light source is configured to provide ultraviolet light in operation and to interrupt providing said ultraviolet light in operation if the sensor detects presence in said space; wherein the emulator device is configured to convey the emulator signal to the sensor; wherein the controller is configured to: control the emulator device to transmit the emulator signal at a first moment in time, determine a verification condition of the sensor if the sensor detects the emulator signal at the first moment in time, determine an error condition of the sensor if the sensor does not detect the emulator signal at the first moment in time, output an output signal upon determining said error condition. Such a lighting device may autonomously verify the correct functioning of the sensor. This is a clear advantage and ensures a lighting device with integrated safety features. In an embodiment, said output signal may be arranged for controlling the lighting device to disable the light source to provide said ultraviolet light in operation. The advantages and/or embodiments applying to the system according to the invention may also apply mutatis mutandis to the lighting device according to the invention.

It is a further object of the invention to provide a method, which at least alleviates the problems and disadvantages mentioned above. Thereto, the invention further provides a method of determining a condition of a sensor of a lighting device; wherein the sensor is configured to detect a presence in a space and to detect an emulator signal, wherein the lighting device comprises a light source configured to provide ultraviolet light in operation and to interrupt providing said ultraviolet light in operation if the sensor detects presence in said space; wherein the method comprises: controlling an emulator device to transmit the emulator signal at a first moment in time; determining a verification condition of the sensor if the sensor detects the emulator signal at the first moment in time, and determining an error condition of the sensor if the sensor does not detect the emulator signal at the first moment in time. The method further comprises (the controller) outputting an output signal upon determining said error condition. The advantages and/or embodiments applying to the system according to the invention may also apply mutatis mutandis to the method according to the invention.

In an embodiment, said output signal may be arranged for controlling the lighting device to disable the light source to provide said ultraviolet light in operation. Hence, the method may comprise the controller controlling the lighting device to disable the light source to provide said ultraviolet light in operation. The output signal may be indicative of the error condition. In an embodiment, the method may comprise the controller outputting the output signal to a further device to notify the further device on the error condition. The space may comprise a surface. In an embodiment, the method may comprise the emulator device projecting the emulator signal onto the surface at the first moment of time.

Aspects of the invention may be implemented in a computer program product, which may be a collection of computer program instructions stored on a computer readable storage device which may be executed by a computer. The instructions of the present invention may be in any interpretable or executable code mechanism, including but not limited to scripts, interpretable programs, dynamic link libraries (DLLs) or Java classes. The instructions can be provided as complete executable programs, partial executable programs, as modifications to existing programs (e.g. updates) or extensions for existing programs (e.g. plugins). Moreover, parts of the processing of the present invention may be distributed over multiple computers or processors

### BRIEF DESCRIPTION OF THE DRAWINGS

The invention will now be further elucidated by means of the schematic non-limiting drawings:
Fig. 1 depicts schematically an embodiment of a system according to the invention;
Fig. 2 depicts schematically an embodiment of a lighting device according to the invention;
Fig. 3 depicts schematically an embodiment of a method according to the invention;
Fig. 4 depicts schematically an embodiment of a system arrangement according to the invention.

### DETAILED DESCRIPTION OF THE EMBODIMENTS

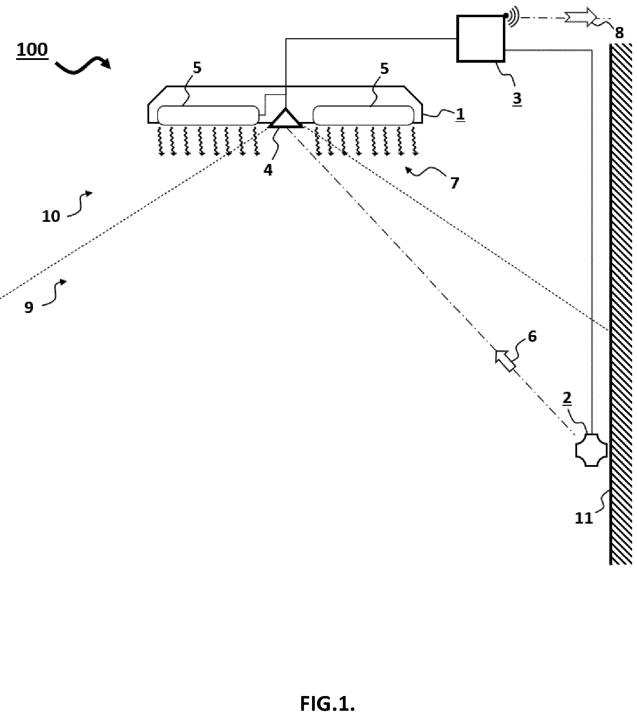

Figure 1 depicts schematically, by non-limiting example, an embodiment of a system 100 according to the invention. The system 100 comprises a lighting device 1, an emulator device 2 and a controller 3. The lighting device 1 comprises a sensor 4 and a light source 5. Both components may be connected, communicate and interact in operation. Here, the sensor 4 is a PIR sensor, but may alternatively be any other type of presence and/or occupancy sensor, such as mentioned in the application, e.g. microwave sensor, light sensor, thermopile sensor, microphone, etc. Here, as an illustrative option, the light source 5 is arranged circularly around the sensor 4. The lighting device 1 is installed in a main space 10. Here, the main space is an office, but may alternatively be a space of a hospital, a retail environment, a warehouse, a car park, a vehicle, a home or a public space. The light source 5 of the lighting device 1 illuminates at least part of the main space 10.

The main space 10 may require an ultraviolet disinfection action, so as to disinfect surfaces and/or fluids (such as air) in the main space 10. The light source 5 of the lighting device 1 therefore provides ultraviolet light 7 in operation. Said in operation may mean during a disinfection action. The disinfection action may e.g. be planned beforehand or scheduled automatically. For convenience, figure 1 depicts the light source 5 providing ultraviolet light 7 as if the light source 5 is in operation.

Since the ultraviolet light 7 may be harmful to humans and/or animals that are present in the main space 10, safety measures ought to be taken. Hence, the PIR sensor 4 of the lighting device 1 is configured to detect presence in the space 9. The space 9 is a part of the main space 10. Hence, the main space 10 comprises the space 9. The space 9 may be considered as the detection region of the sensor 4.

Thereby, the lighting device 1, more particularly the light source 5 of the lighting device 1, will interrupt providing said ultraviolet light 7 in operation when the sensor 4 detects a presence in said space 9. The PIR sensor 4 is therefore an important feature for safety. Hence, the PIR sensor 4 ought to be checked e.g. regularly on whether it is still functioning correctly.

Therefore, the system 100 provides the emulator device 2 and the controller 3. The emulator device 2 is arranged in the space 9. The emulator device 2 is an infrared light source. The emulator device 2 is configured to convey an emulator signal 6 to the PIR sensor 4. Here, the emulator signal 6 is an infrared light signal 6. The emulator signal 6 is thereby detectable by the PIR sensor 4. The emulator device may alternatively be arranged in the main space and project an emulator signal on a surface in the space.

The emulator device may e.g. be battery powered. Here, the emulator device 2 is mounted to a wall 11 in the space 9. The wall 11 is thereby a delineating surface that delineates the space 9, and delineates similarly the main space 10. The wall may alternatively be any other delineating surface, such as mentioned in the application. Here, the distance between the emulator device 2 is five meters from the sensor 4, but alternatively the emulator device may be at least two, at least four or at least eight meters from the sensor. This distance may depend on the UV light source, the lighting characteristic of the UV illumination, a sensor type of the sensor, etc. Thus, the distance between the emulator device and the sensor covers a significant part of the space 9, because presence of people and/or animals may also be detected within said range of five meters from the sensor 4. In alternative examples, the emulator device may also be comprised by another device, such as an electric device, e.g. a switch, a portable user device, a lighting device.

Still referring to figure 1, as mentioned, the system also comprises the controller 3. The controller 3 is arranged separately from the lighting device 1 and the emulator device 2. Alternatively, the lighting device and/or the emulator device, or yet another electric device, may comprise the controller. Here, the controller 3 communicates with the lighting device 1 and the emulator device 2 via a wired connection, whereas said communication may alternatively be via wireless communication such as e.g. Bluetooth, ZigBee, Wi-Fi, RF, IR, Lo-Ra, UWB, 5G, Li-Fi, VLC, etc. Here, the controller 3 is arranged in the main space 10, but alternatively the controller may be arranged outside said space in another space.

The controller 3 controls the emulator device 2 to transmit the emulator signal 6 (i.e. the infrared light signal) at a first moment in time. The emulator signal 6 is thereby detectable for a correct functioning PIR sensor 4. The controller 3 is also configured to determine a verification condition of the sensor 4 (i.e. a condition that verifies the correct functioning of the sensor), if the sensor 4 detects the emulator signal 6 at the first moment in time. Similarly, the controller 3 is also configured to determine an error condition of the sensor 4 (i.e. a condition that confirms the malfunctioning of the sensor), if the sensor 4 does not detect the emulator signal 6 at the first moment in time. The first moment in time may thereby be at least one first moment in time, i.e. the verification of the sensor 4 may be performed more often.

Upon determining the verification condition, the controller 3 does not provide any further action. In alternative examples tough, the controller may output a verification signal upon determining said verification signal. Said verification signal may be indicative of the verification condition. Said verification signal may be arranged to control a further electric device (such as e.g. a safety indicator light), and/or to store an entry indicative of said verification in a memory, a server, or management system.

Upon determining the error condition, the controller 3 outputs an output signal 8. Here, the output signal 8 is indicative of the error condition, and the controller 3 wirelessly conveys the output signal 8 to a further device (not depicted). The further device is a building management device. Because of the building management device obtaining the output signal indicative of the error condition, the building management device is notified on the error condition. The error condition may thereby be stored or logged in a memory of the building management device. This enables safety. The building management device may e.g. send out a repair request for the lighting device 1 and the corresponding malfunctioning sensor 4, or inform a building manager about this. Alternative examples, which may comprise other types of further devices, as mentioned before in this application, may be envisioned similarly.

In an embodiment (which is not explicitly depicted but related to the embodiment depicted in figure 1), additionally or alternatively, an output signal may be arranged for controlling the lighting device to disable the light source to provide said ultraviolet light in operation. Therefore, upon determining that the sensor may not be functioning accordingly (in detecting the presence), the lighting device may be disabled, or the light source may be disabled to provide said ultraviolet light in operation. This advantageously prevents a risk of operating the light source to provide ultraviolet light while presence in the space may not be detected accordingly.

All in all, the emulator signal 6 is thus purposively transmitted to the sensor 4 at the first moment in time. The emulator device 2 may thus emulate the presence in the space 9. This transmission may advantageously be used to determine a condition of the sensor 4 of the lighting device 1. Namely, the controller 3 is configured to determine a verification condition of the sensor 4 if the sensor 4 detects the emulator signal 6 at that first moment in time. The sensor 4 is thereby tested accordingly. A building management device is also advantageously notified, and additionally or alternatively, the lighting device 1 may be disabled and/or the light source 5 may be disabled to provide said ultraviolet light 7. Therefore, the system 100 according to the invention is able to verify the correct working or functioning of the sensor 4 (by determining said verification condition) and act in case of malfunctioning.

Still referring to figure 1, the ultraviolet light of the light source may for example comprise UV-A (315-400 nm), UV-B (280-315 nm), UV-C (100-280 nm), Far-UV, Extreme-UV. More specifically, in aspects, said spectral distribution may comprise at least one of: Ultra Deep UV in the range of 100 -190 nm, Deep UV light in the range of 190-220 nm, UV-C light in the range of 220-280 nm, UV-B light in the range of 280-315 nm, UV-A light in the range of 315-400 nm, UV light at 254 nm. Such an ultraviolet spectrum of light may have proven effectively in killing infectious matter, such as viruses, bacteria, fungi, yeasts and/or germs. For example, UV-C lighting comprises a strong bactericidal effect, damaging the structure of DNA in living cells.

The light source may be phrased as lighting unit or lighting module. Said lighting unit or lighting module may comprise light emitting diodes (LEDs), a chips-on-board (COB) light source (the term "COB" especially refers to LED chips in the form of a semiconductor chip that is neither encased nor connected but directly mounted onto a substrate, such as a PCB) and/or a laser. The term "laser" especially refers to a device that emits light through a process of optical amplification based on the stimulated emission of electromagnetic radiation. Especially, in embodiments the term "laser" may refer to a solid-state laser. In specific embodiments, the terms "laser" or "laser light source", or similar terms, refer to a laser diode (or diode laser).

The light source may be a collimated source of light, a point source of light, or an elongated source of light. The disinfection device may comprise an optical arrangement, wherein the light source may comprise said optical arrangement, wherein an optical setting of the optical arrangement may be set so as to perform a disinfection action. The optical setting may e.g. comprise an intensity and/or the spatial distribution of a light beam generated by the light source.

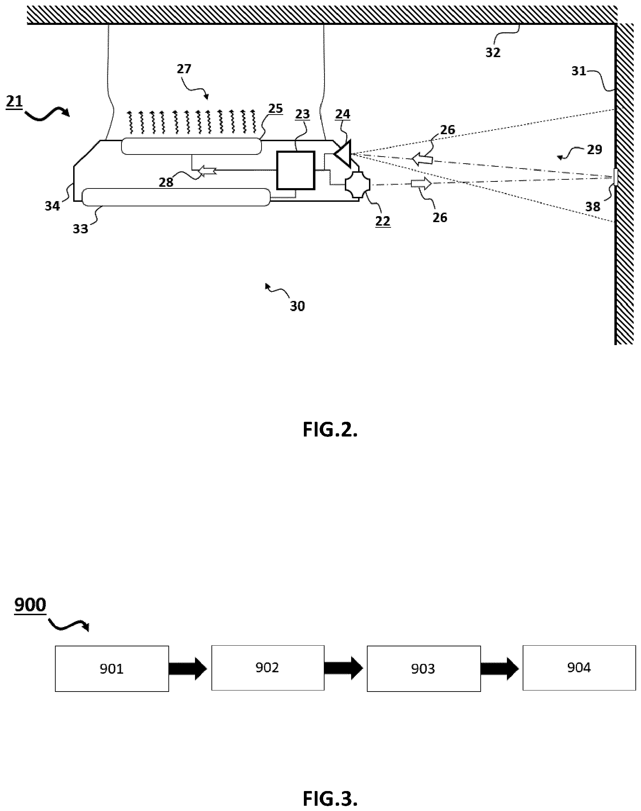

Figure 2 depicts schematically, by non-limiting example, an embodiment of a lighting device 21 according to the invention. The lighting device 21 may be part of a system or a system arrangement according to the invention. The lighting device 21 may also be considered a system according to the invention.

The lighting device 21 is mounted within a main space 30. Here, the main space 30 is a ward in a hospital, but may alternatively be any other space requiring disinfection, such as e.g. an office, a stadium, a shop, or an animal farm. Here, the lighting device 21 is a luminaire. The luminaire is suspended from a ceiling 32 of the main space 30. Thus, the luminaire 21 is hanging from the ceiling 32 due to gravity. The main space 30 and the space 29 are further delineated by a wall 31.

The lighting device 21 (i.e. the suspended luminaire) comprises an emulator device 22, a controller 23, a sensor 24, a light source 25, and optionally a further light source 33. More specially, the lighting device 21 comprises a luminaire housing 34 comprising (or: housing, or: accommodating) said emulator device 22, controller 21, sensor 24, light source 25 and optionally further light source 33. All these components may be connected with each other via the controller 23, and communicate therewith. The optionally further light source 33 may in operation provide functional or ambient light to the main space 30.

The light source 25 provides ultraviolet light 27 in operation, so as to disinfect a surface and/or fluid in the main space 30. Said in operation may mean during a disinfection action. The light source 25 may optionally comprise optics to control a lighting characteristic of the light source. Here, the light source 25 provides the ultraviolet light 27 in a direction opposite to gravity, i.e. towards the ceiling 32. The light source 25 therefore illuminates at least part of the main space 30, namely a part of the ceiling 32 of the main space, and the upper air in the main space 30. The lighting device 21 may thus be considered an upper air UV-disinfecting luminaire.

Since the ultraviolet light 27 may be harmful to humans and/or animals that are present in the main space 30, i.e. the hospital ward, safety measures ought to be taken. Hence, the sensor 24 of the lighting device 21 is configured to detect presence in the space 29 comprised by the main space 30. Here, the sensor 24 is a PIR sensor, but may alternatively be any other type of presence and/or occupancy sensor, such as mentioned in the application, e.g. microwave sensor, light sensor, thermopile, microphone, etc.

Here, the sensor 24 has a narrow detection region, and monitors the space 29 between the suspended luminaire 21 and the wall 31. The sensor 24 can therefore detect any protrusion through said space 29, for example detecting the presence of any body part, such as a head or a hand protruding in said space 29. This may pose a safety risk. The PIR sensor 24 is therefore an important feature for safety. Hence, the PIR sensor 24 ought to be checked e.g. regularly on whether it is still functioning correctly. Alternatively, other configurations of the sensor may be envisioned, such as the PIR sensor monitoring at least part of the space beneath the lighting device with a wide angle for presence, etc.

Referring to figure 2, the controller 23 of the lighting device 21 will interrupt the light source 25 to provide said ultraviolet light 7 in operation when the PIR sensor 24 detects a presence in said space 29. The lighting device 21 further comprises the emulator device 22. The emulator device 22 is arranged in the lighting device and conveys an emulator signal 26 to the PIR sensor 24.

Namely, the emulator device 22 is an infrared laser, and the emulator signal 26 is an infrared laser signal 26. The infrared laser 22 projects the infrared laser signal 26 onto a projection location 38 on the wall 31. This projection location 38 is in the space 29 and may thus be detected by the PIR sensor 24. The emulator signal 26, i.e. the infrared laser signal, is thereby at least partly reflected on said projection location 38 of the wall 31 and propagates to the PIR sensor 24. The distance that the emulator signal propagates, that is the propagation distance that the emulator signal 26 propagates through the main space 31 between the emulator device 22 and the sensor 24, may thereby be at least two meters.

The emulator signal 26 is thereby detectable for a correct functioning PIR sensor 24. Namely, the controller 23 controls the emulator device 22 to transmit the emulator signal 26 at a first moment in time. The controller 23 is also configured to determine a verification condition of the sensor 24 (i.e. a condition that verifies the correct functioning of the sensor), if the sensor 24 detects the emulator signal 26 at the first moment in time. Similarly, the controller 23 is also configured to determine an error condition of the sensor 24 (i.e. a condition that confirms the malfunctioning of the sensor), if the sensor 24 does not detect the emulator signal 26 at the first moment in time. The first moment in time may thereby be at least one first moment in time, i.e. the verification of the sensor 24 may be performed more often. The lighting device 21 may thus autonomously verify the correct functioning of the sensor 24. This is a clear advantage and ensures a lighting device 21 with integrated safety features.

Upon determining the verification condition, in this embodiment, the controller 23 does not provide any further action. Alternatively, the controller may store, or log said verification condition. Yet alternatively, the controller may inform another device that the lighting device is operating with a functioning sensor.

Upon determining the error condition, the controller 23 outputs an output signal 28. Here, the output signal 28 is a control signal, because said output signal is arranged for controlling the lighting device 21 to disable the light source 25 to provide said ultraviolet light 27 in operation. More specifically, the controller disables the light source 25 to provide said ultraviolet light 27 in operation upon determining said error condition.

Other examples of the controller acting upon determining the error condition may be envisioned similarly. In examples, the controller may also control the further light source 33 to illuminate the main space with a lighting characteristic upon determining said error condition. The lighting characteristic may e.g. be at least one of a modulation, a light pattern, an intensity, a color, etc. For example, blinking red light. In examples: Upon determining the error condition, the controller may output an output signal indicative of the error condition, and the controller may e.g. wirelessly convey the output signal to a further device. The further device may e.g. be a user device, a server, a smartphone, a wearable device, a luminaire repair database, etc.

In an embodiment, not depicted, the system according to figure 1 and/or the lighting device according to figure 2 is provided, but wherein the light source is arranged for purifying air with the ultraviolet light. Therefore, the lighting device comprises a purifier for purifying air in operation, wherein the purifier comprises said light source. The light source may thus not be directly visible from the main space, but still pose a risk to any animal and/or person present. The present invention may thus provide a safety check for the sensor and the lighting device with the use of the emulator device.

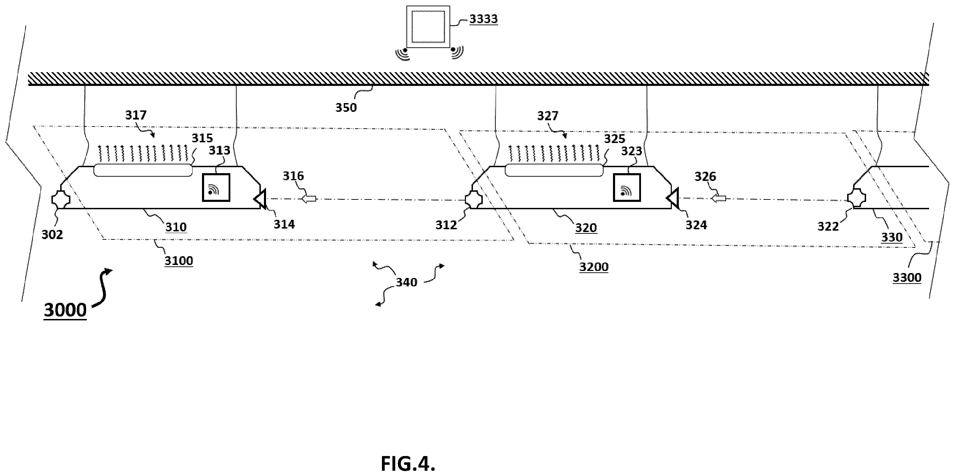

Figure 4 depicts schematically, by non-limiting example, an embodiment of a system arrangement 3000 according to the invention. The system arrangement 3000 comprises a plurality of systems or lighting devices according to the invention and a main controller 3333. The system arrangement 3000 is installed in a main space 340. The main space 340 comprises a surface 350, which surface is a ceiling 350. Here, the controller is arranged outside the main space 340, but may alternatively be arranged within the main space

For convenience, as depicted in figure 4, the system arrangement 3000 comprises three systems 3100, 3200, 3300 according to the invention. These systems 3100, 3200, 3300 are now referred to as lighting systems 3100, 3200, 3300, because they comprise a respective lighting device 310, 320, 330. This plurality of systems may be arranged in a grid pattern, which is depicted in the present figure two-dimensionally as a 3x1 matrix configuration. The third lighting system 3300 is only partly depicted. The second lighting system 3200 is adjacent to the first lighting system 3100 and the third lighting system 3300. The lighting systems may comprise further lighting systems similar to these three lighting systems 3100, 3200, 3300. Hence, the lighting system arrangement may compromise any other number of lighting systems, such as at least 4, at least 5, at least 8, etc.

The first lighting system 3100 comprises a first lighting device 310 and a second lighting device 320. The first lighting device 310 and the second lighting device 320 are similarly suspended from the ceiling 350. The first lighting system 3100 also comprises an emulator device 312, a controller 313, a sensor 314 and a light source 315. Thereby, the first lighting device 310 comprises the sensor 314, the controller 313 and the light source 315. These components may be connected, communicate and interact in operation, e.g. via the controller 323. Thereby, the second lighting device 320 comprises the emulator device 312 of the first lighting device 310. Thereby, the first lighting device 320 further comprises an emulator device 302 of another adjacent lighting device (not depicted). This emulator device of another adjacent lighting device may be optional for the first lighting device though.

Alternatively phrased: The first lighting system may comprise the first lighting device and the emulator device. Thereby, the first lighting device may comprise the controller, the sensor, and the light source. The emulator device of the first lighting system may be arranged remotely from the first lighting device. For example, the emulator device may be comprised in a further apparatus. Said further apparatus may thus be the second lighting device of the second lighting system.

Still referring to figure 4, the system arrangement 3000 also comprises the second lighting system 3200. The second lighting system 3200 comprises the second lighting device 320 and a third lighting device 330. The second lighting system 3200 also comprises an emulator device 322, a controller 323, a sensor 324 and a light source 325. Thereby, the second lighting device 320 comprises the sensor 324, the controller 323 and the light source 325. These components may be connected, communicate and interact in operation, e.g. via the controller 323. Thereby, the third lighting device 330 comprises the emulator device 322 of the second lighting device 320. As mentioned, in the depicted embodiment, the second lighting device 320 further comprises the emulator device 312 of the first lighting device 310 of the first lighting system 3100.

Alternatively phrased: The second lighting system may comprise the second lighting device and the emulator device. Thereby, the second lighting device may comprise the controller, the sensor, and the light source. The emulator device of the second lighting system may be arranged remotely from the second lighting device. For example, the emulator device may be comprised in a further apparatus. Said further apparatus may thus be the third lighting device of the third lighting system.

The system arrangement 3000 also comprises the third lighting system 3300. The third lighting system 3300 comprises the third lighting device 330, which third lighting device 330 comprises the emulator device 322 of the second lighting system 3200. The second lighting device 320 and the third lighting device 330 are also similarly suspended from the ceiling 350. The third lighting device 330 may further comprise, mutatis mutandis, the same features as the first lighting device and/or the second lighting device. The first lighting system 3100, the second lighting system 3200, and the third lighting system 3300 may thus be identical, and be indicative for a number of repetitive lighting systems of the system arrangement 3000.

Still referring to figure 4, the light source 315 of the first lighting device 310 and the light source 325 of the second lighting device 320 provide ultraviolet light 317, 327 in operation. Said in operation may be during a disinfection action. The controller 313 of the first lighting device 310 may thereby control, or convey a control signal to, the light source 315 of the first lighting device 310. The controller 323 of the second lighting device 320 may thereby control, or convey a control signal to, the light source 325 of the second lighting device 320. The light source 315 of the first lighting device 310 and the light source 325 of the second light source 325 may be controlled independently and/or separately, yet alternatively in coordination with each other.

Therefore, the sensor 314 of the first lighting device 310 is configured to detect presence in a first space (not explicitly depicted). The sensor 314 may be a presence sensor as mentioned in this application. The first space may be considered the detection region of the sensor 314 of the first lighting device 310. The main space 340 comprises this space. Moreover, the light source 315 of the first lighting device 310 is configured to interrupt providing said ultraviolet light 317 in operation when the sensor 314 of the first lighting device 310 detects presence in said first space.

Therefore, the sensor 324 of the second lighting device 320 is configured to detect presence in a second space (not explicitly depicted). The sensor 324 may be a presence sensor as mentioned in this application. The second space may be considered the detection region of the sensor 324 of the second lighting device 320. The main space 340 comprises this space. Moreover, the light source 325 of the second lighting device 320 is configured to interrupt providing said ultraviolet light 327 in operation when the sensor 324 of the second lighting device 320 detects presence in said second space.

Since ultraviolet light 317, 327 may be dangerous to humans and/or animals, and because the safety is ensured respectively by the sensor 314 of the of the first lighting device 310 and the sensor 324 of the of the second lighting device 320, these sensors 314, 324 ought to be checked e.g. regularly on whether they are still functioning correctly.

This is done by the emulator devices as mentioned above. Namely, the emulator device 312 of the first lighting system 3100, which is comprised by the second lighting device 320, is arranged to convey a first emulator signal 316 to the sensor 314 of the first lighting system 3100, which is comprised by the first lighting device 310. Namely, the emulator device 322 of the second lighting system 3200, which is comprised by the third lighting device 330, is arranged to convey a second emulator signal 326 to the sensor 324 of the second lighting system 3200, which is comprised by the second lighting device 320.

Moreover, the main controller 3333 of the system arrangement 3000 comprises the controller 313 of the first lighting device 310, the controller 314 of the second lighting device, and the controller (not depicted) of the third lighting device 330. The main controller 3333 is able to control at least one respective emulator device of a respective lighting system of the plurality of lighting systems depicted in figure 4, which control may be via the respective controllers in the corresponding lighting devices. The main controller 3333 thereby communicates wirelessly with the respective controllers in the corresponding lighting devices, but may alternatively communicate via a wired connection.

More specifically: the main controller 3333 controls the emulator device 312 of the first lighting system 3100 to transmit the first emulator signal 316 at a first moment in time to the sensor 314 of the first lighting device 310. The distance between the emulator device 312 and the sensor 314 of the first lighting system 3100 may be at least two meters. Further: The main controller 3333 determines a first verification condition the sensor 314 of the first lighting device 310, if this sensor 314 detects the first emulator signal 316 at the first moment in time. The main controller 3333 determines a first error condition the sensor 314 of the first lighting device 310, if this sensor 314 does not detect the first emulator signal 316 at the first moment in time.

Similarly: the main controller 3333 controls the emulator device 322 of the second lighting system 3200 to transmit the second emulator signal 326 at a second moment in time to the sensor 324 of the second lighting device 320. The distance between the emulator device 322 and the sensor 324 of the second lighting system 3200 may be at least two meters. Further: The main controller 3333 determines a second verification condition the sensor 324 of the second lighting device 320, if this sensor 324 detects the second emulator signal 326 at the second moment in time. The main controller 3333 determines a second error condition the sensor 324 of the second lighting device 320, if this sensor 324 does not detect the second emulator signal 326 at the second moment in time.

The first lighting system 3100, the second lighting system 3200, and the third lighting system 3300 are identical. Hence, the above may mutatis mutandis apply to the third lighting system, and the emulator device and sensor thereof.

Alternatively, said transmitting of the emulator signal 316, 326 may be done indirectly. That is: The sensor of the first lighting device may detect presence in a space comprising a surface, wherein the emulator device may project the respective emulator signal onto a projection location on the surface (being monitored by the sensor of the first lighting device), so as to convey the respective emulator signal to the sensor of the first lighting device. The same may mutatis mutandis apply for the sensor of the second lighting device.

Still referring to figure 4, the system arrangement 3000 comprises the main controller 3333 so as to provide a centralized system arrangement 3000 that centrally controls the plurality of lighting systems of the system arrangement 3000. Alternatively, the controller of each respective lighting system of the system arrangement may perform the verification action of the main controller at least partly locally and/or in a distributed way. Thus, the system arrangement may e.g. comprise a plurality of embodiments as depicted in figure 1, wherein the emulator device is not mounted on a wall, but is comprised by an adjacent lighting device.

Furthermore, upon the main controller 3333 determining the first verification condition, the main controller 3333 may not provide any further action; and/or upon the main controller 3333 determining the second verification condition, the main controller 3333 may not provide any further action. Alternatively, for both situations, the controller may store, or log said verification condition. Yet alternatively, the main controller may inform another device that the first lighting device is operating with a functioning sensor.

However, upon determining the first error condition and/or the second error condition, the main controller 3333 outputs an output signal. The output signal is indicative of the first error condition and/or the second error condition. The main controller 3333 transmits this output signal to a further device. This further device may e.g. be a smartphone of a lighting system manager, a mechanic, or a venue owner. The output signal may also be transmitted to a server, computer, or building management device; for example, to be stored or logged in a memory thereof.

Here, upon determining the first error condition, the main controller 3333 also transmits a control signal to the first lighting device 310, e.g. to the controller 313 of the first lighting device 310, so as to temporarily disable the light source 315 of the first lighting device 310 in providing ultraviolet light 317 in operation. The light source 315 may for example still provide other, less harmful, light spectra.

Here, upon determining the second error condition, the main controller 3333 also transmits a control signal to the second lighting device 320, e.g. to the controller 323 of the second lighting device 320, so as to temporarily disable the light source 325 of the first lighting device 320 in providing ultraviolet light 327 in operation. The light source 325 may for example still provide other, less harmful, light spectra.

All in all, the main controller 3334 coordinates the purposively transmitting of the first and/or second emulator signal at the respective first and second moment in time. Said first moment in time may be a plurality of first moments in time. Said second moment in time may be a plurality of second moments in time.

The emulator devices may thus emulate the presence in the spaces monitored by the sensors. This enables the main controller to determine a verification condition of the sensors. The sensors are thereby tested accordingly. Due to the spatially organized positioning of the lighting devices in the system arrangement, each lighting device may advantageously facilitate the verification of the sensor of an adjacent lighting device. This is further facilitated by the insight that lighting devices in a main space often have the installation height. The main controller also advantageously takes suitable action when a dangerous error condition is determined.

Figure 3 depicts schematically, by non-limiting example, an embodiment of a method 900 according to the invention. The method 900 may be performed by a lighting device, and/or a lighting system according to the invention. Hence, the method 900 describes determining a condition of a sensor of a lighting device, wherein the sensor is configured to detect a presence in a space and/or to detect an emulator signal, wherein the lighting device comprises a light source configured to provide ultraviolet light in operation and to interrupt providing said ultraviolet light in operation if the sensor detects presence in said space. The method 900 comprises an initial step 901 of controlling an emulator device to transmit an emulator signal at a first moment in time. The method comprises a step 902 of determining a verification condition of a sensor if the sensor detects the emulator signal at the first moment in time, and a step 903 of determining an error condition of the sensor if the sensor does not detect the emulator signal at the first moment in time. The method may further comprise a step 904 of outputting an output signal upon determining said error condition. Said output signal may be arranged for controlling the lighting device to disable the light source to provide said ultraviolet light in operation. Hence, step 904 may be the controller disabling the light source in providing said ultraviolet light in operation. Said output signal may in examples be a notification signal, or an entry to be stored in a further device.

## Claims

1. A system (100) comprising:
- a lighting device (1, 21) comprising a sensor (4) and a light source (5), wherein the sensor (4) is configured to detect a presence in a space (10) and to detect an emulator signal (6),
- an emulator device (2) configured to convey the emulator signal (6) to the sensor (4);
- a controller (3) configured to control the emulator device (2) to transmit the emulator signal (6) at a first moment in time, and to determine a verification condition of the sensor (4) if the sensor (4) detects the emulator signal (6) at the first moment in time;
and **characterised in that**,
the light source (5) is configured to provide ultraviolet light (7) in operation and to interrupt providing said ultraviolet light (7) in operation when the sensor (4) detects presence in said space (10).

2. The system according to claim 1, wherein the controller (3) is configured to determine an error condition of the sensor (4) if the sensor (4) does not detect the emulator signal (6) at the first moment in time; wherein the controller (3) is configured to output an output signal (8) upon determining said error condition.

3. The system according to claim 2, wherein said output signal (8) is arranged for controlling the lighting device (1) to disable the light source (5) to provide said ultraviolet light (7) in operation.

4. The system according to claim 2, wherein said output signal (8) is indicative of the error condition, wherein the controller (3) is configured to output the output signal (8) to a further device so as to notify the further device on the error condition.

5. The system according to any one of the preceding claims,
wherein the sensor (4) is a PIR sensor, the emulator device (2) is an infrared light source, and the emulator signal (6) is an infrared signal; or
wherein the sensor (4) is microwave sensor, the emulator device (2) is a radiofrequency beacon, and the emulator signal (6) is a radiofrequency signal; or
wherein the sensor (4) is a light sensor, and the emulator device (2) is a light beacon, wherein the emulator signal (6) is a light signal

6. The system according to any one of the preceding claims, wherein the light source (5) is configured to illuminate in operation at least part of the space (9) with the ultraviolet flight (7), or to illuminate at least part of a main space (10) comprising the space (9) with the ultraviolet light (7).

7. The system according to any one of the preceding claims, wherein the lighting device (1, 21) comprises a purifier for purifying air in operation, wherein the purifier comprises the light source (5), wherein the ultraviolet light (7) of the light source (5) purifies the air.

8. The system according to any one of the preceding claims, wherein the lighting device (21) comprises the controller (23).

9. The system according to any one of the preceding claims, wherein the space (29) comprises a surface (38), wherein the emulator device (32) is configured to project said emulator signal (26) onto the surface (38).

10. The system according to any one of the preceding claims, wherein the emulator device (2) is arranged at a distance from the sensor (4), wherein said distance is at least two meters.

11. The system according to any one of the preceding claims, wherein the system (3000) comprises a second lighting device (3200); wherein the second lighting device (3200) comprises the emulator device (312).

12. The system according to any one of the preceding claims 1-9, wherein the lighting device (21) comprises the emulator device (22).

13. The system according to any one of the preceding claims, wherein the lighting device (21) is a luminaire and comprises a luminaire housing (34), wherein the luminaire housing (34) comprises the sensor (24), the light source (25), the controller (23) and the emulator device (22).

14. A system arrangement comprising a plurality of systems (3100, 3200, 3300) according to any one of the preceding claims 1-11, wherein the respective lighting device of at least one system of the plurality of systems (3100, 3200, 3300) comprises the respective emulator device of one other system of the plurality of systems (3100, 3200, 3300).

15. A method (900) of determining a condition of a sensor (4) of a lighting device (1, 21);
wherein the sensor (4) is configured to detect a presence in a space (9) and to detect an emulator signal (6),
wherein the method (900) comprises:
- controlling (901) an emulator device (2) to transmit the emulator signal (6) at a first moment in time;
- determining (902) a verification condition of the sensor (4) if the sensor (4) detects the emulator signal (6) at the first moment in time, and determining (902) an error condition of the sensor (4) if the sensor (4) does not detect the emulator signal (6) at the first moment in time;
- outputting (903) an output signal (8) upon determining said error condition;
**characterised in that**,
the lighting device (1, 21) comprises a light source (5) configured to provide ultraviolet light (7) in operation and to interrupt providing said ultraviolet light (7) in operation if the sensor (4) detects presence in said space (10).

## Patentansprüche

1. System (100), umfassend:
- eine Beleuchtungsvorrichtung (1, 21), umfassend einen Sensor (4) und eine Lichtquelle (5), wobei der Sensor (4) konfiguriert ist, um eine Präsenz in einem Raum (10) zu erkennen und ein Emulatorsignal (6) zu erkennen,
- eine Emulatorvorrichtung (2), die konfiguriert ist, um das Emulatorsignal (6) an den Sensor (4) zu übermitteln;
- eine Steuerung (3), die konfiguriert ist, um die Emulatorvorrichtung (2) zu steuern, um das Emulatorsignal (6) zu einem ersten Zeitpunkt zu übertragen, und um eine Verifizierungsbedingung des Sensors (4) zu bestimmen, wenn der Sensor (4) das Emulatorsignal (6) zu dem ersten Zeitpunkt erkennt;
und **dadurch gekennzeichnet, dass,**
die Lichtquelle (5) konfiguriert ist, um ultraviolettes Licht (7) im Betrieb bereitzustellen und das Bereitstellen des ultravioletten Lichts (7) im Betrieb zu unterbrechen, wenn der Sensor (4) die Präsenz in dem Raum (10) erkennt.

2. System nach Anspruch 1, wobei die Steuerung (3) konfiguriert ist, um einen Fehlerzustand des Sensors (4) zu bestimmen, wenn der Sensor (4) das Emulatorsignal (6) zum ersten Zeitpunkt nicht erkennt;
wobei die Steuerung (3) konfiguriert ist, um ein Ausgangssignal (8) beim Bestimmen des Fehlerzustands auszugeben.

3. System nach Anspruch 2, wobei das Ausgangssignal (8) zum Steuern der Beleuchtungsvorrichtung (1) angeordnet ist, um die Lichtquelle (5) zu deaktivieren, um das ultraviolette Licht (7) in Betrieb bereitzustellen.

4. System nach Anspruch 2, wobei das Ausgangssignal (8) den Fehlerzustand anzeigt, wobei die Steuerung (3) konfiguriert ist, um das Ausgangssignal (8) an eine weitere Vorrichtung auszugeben, um die weitere Vorrichtung über den Fehlerzustand zu benachrichtigen.

5. System nach einem der vorstehenden Ansprüche,
wobei der Sensor (4) ein PIR-Sensor ist, die Emulatorvorrichtung (2) eine Infrarot-Lichtquelle ist, und das Emulatorsignal (6) ein Infrarot-Signal ist; oder
wobei der Sensor (4) ein Mikrowellensensor ist, die Emulatorvorrichtung (2) eine Hochfrequenzbake ist und das Emulatorsignal (6) ein Hochfrequenzsignal ist; oder
wobei der Sensor (4) ein Lichtsensor ist, und die Emulatorvorrichtung (2) eine Lichtbake ist,
wobei das Emulatorsignal (6) ein Lichtsignal ist.

6. System nach einem der vorstehenden Ansprüche, wobei die Lichtquelle (5) konfiguriert ist, um im Betrieb mindestens einen Teil des Raums (9) mit dem ultravioletten Licht (7) zu beleuchten, oder mindestens einen Teil eines Hauptraums (10) zu beleuchten, umfassend den Raum (9) mit dem ultravioletten Licht (7).

7. System nach einem der vorstehenden Ansprüche, wobei die Beleuchtungsvorrichtung (1, 21) einen Reiniger zum Reinigen von Luft im Betrieb umfasst, wobei der Reiniger die Lichtquelle (5) umfasst, wobei das ultraviolette Licht (7) der Lichtquelle (5) die Luft reinigt.

8. System nach einem der vorstehenden Ansprüche, wobei die Beleuchtungsvorrichtung (21) die Steuerung (23) umfasst.

9. System nach einem der vorstehenden Ansprüche, wobei der Raum (29) eine Oberfläche (38) umfasst, wobei die Emulatorvorrichtung (32) konfiguriert ist, um das Emulatorsignal (26) auf die Oberfläche (38) zu projizieren.

10. System nach einem der vorstehenden Ansprüche, wobei die Emulatorvorrichtung (2) in einem Abstand von dem Sensor (4) angeordnet ist, wobei der Abstand mindestens zwei Meter beträgt.

11. System nach einem der vorstehenden Ansprüche, wobei das System
(3000) eine zweite Beleuchtungsvorrichtung (3200) umfasst;
wobei die zweite Beleuchtungsvorrichtung (3200) die Emulatorvorrichtung (312) umfasst.

12. System nach einem der vorstehenden Ansprüche 1 bis 9, wobei die Beleuchtungsvorrichtung (21) die Emulatorvorrichtung (22) umfasst.

13. System nach einem der vorstehenden Ansprüche, wobei die Beleuchtungsvorrichtung (21) eine Leuchte ist und ein Leuchtengehäuse (34) umfasst, wobei das Leuchtengehäuse (34) den Sensor (24), die Lichtquelle (25), die Steuerung (23) und die Emulatorvorrichtung (22) umfasst.

14. Systemanordnung, umfassend eine Vielzahl von Systemen (3100, 3200, 3300) nach einem der vorstehenden Ansprüche 1 bis 11, wobei die jeweilige Beleuchtungsvorrichtung von mindestens einem System der Vielzahl von Systemen (3100, 3200, 3300) die jeweilige Emulatorvorrichtung eines anderen Systems der Vielzahl von Systemen (3100, 3200, 3300) umfasst.

15. Verfahren (900) zum Bestimmen eines Zustands eines Sensors (4) einer Beleuchtungsvorrichtung (1, 21);
wobei der Sensor (4) konfiguriert ist, um eine Präsenz in einem Raum (9) zu erkennen und ein Emulatorsignal (6) zu erkennen,
wobei das Verfahren (900) umfasst:
- Steuern (901) einer Emulatorvorrichtung (2), um das Emulatorsignal (6) zu einem ersten Zeitpunkt zu übertragen;
- Bestimmen (902) einer Verifizierungsbedingung des Sensors (4), wenn der Sensor (4) das Emulatorsignal (6) zu dem ersten Zeitpunkt erkennt, und Bestimmen (902) eines Fehlerzustands des Sensors (4), wenn der Sensor (4) das Emulatorsignal (6) zu dem ersten Zeitpunkt nicht erkennt;
- Ausgeben (903) eines Ausgangssignals (8) beim Bestimmen des Fehlerzustands;
**dadurch gekennzeichnet, dass**
die Beleuchtungsvorrichtung (1, 21) eine Lichtquelle (5) umfasst, die konfiguriert ist, um ultraviolettes Licht (7) im Betrieb bereitzustellen und um das Bereitstellen des ultravioletten Lichts (7) im Betrieb zu unterbrechen, wenn der Sensor (4) eine Präsenz in dem Raum (10) erkennt.

## Revendications

1. Système (100) comprenant :
- un dispositif d'éclairage (1, 21) comprenant un capteur (4) et une source lumineuse (5), dans lequel le capteur (4) est configuré pour détecter une présence dans un espace (10) et pour détecter un signal d'émulation (6),
- un dispositif d'émulation (2) configuré pour transmettre le signal d'émulation (6) au capteur (4) ;
- un dispositif de commande (3) configuré pour commander le dispositif d'émulation (2) pour transmettre le signal d'émulation (6) à un premier instant, et pour déterminer une condition de vérification du capteur (4) si le capteur (4) détecte le signal d'émulation (6) au premier instant ;
**et caractérisé en ce que,**
la source lumineuse (5) est configurée pour fournir de la lumière ultraviolette (7) en fonctionnement et pour interrompre la fourniture de ladite lumière ultraviolette (7) en fonctionnement lorsque le capteur (4) détecte une présence dans ledit espace (10).

2. Système selon la revendication 1, dans lequel le dispositif de commande (3) est configuré pour déterminer une condition d'erreur du capteur (4) si le capteur (4) ne détecte pas le signal d'émulation (6) au premier instant ;
dans lequel le dispositif de commande (3) est configuré pour fournir un signal de sortie (8) lors de la détermination de ladite condition d'erreur.

3. Système selon la revendication 2, dans lequel ledit signal de sortie (8) est agencé pour commander le dispositif d'éclairage (1) afin de désactiver la source lumineuse (5) pour fournir ladite lumière ultraviolette (7) en fonctionnement.

4. Système selon la revendication 2, dans lequel ledit signal de sortie (8) indique la condition d'erreur, dans lequel le dispositif de commande (3) est configuré pour fournir le signal de sortie (8) à un autre dispositif afin d'informer l'autre dispositif de la condition d'erreur.

5. Système selon l'une quelconque des revendications précédentes,
dans lequel le capteur (4) est un capteur PIR, le dispositif d'émulation (2) est une source de lumière infrarouge, et le signal d'émulation (6) est un signal infrarouge ; ou
dans lequel le capteur (4) est un capteur hyperfréquence, le dispositif d'émulation (2) est une balise radiofréquence, et le signal d'émulation (6) est un signal radiofréquence ; ou
dans lequel le capteur (4) est un capteur de lumière, et le dispositif d'émulation (2) est une balise lumineuse,
dans lequel le signal d'émulation (6) est un signal lumineux.

6. Système selon l'une quelconque des revendications précédentes, dans lequel la source lumineuse (5) est configurée pour éclairer en fonctionnement au moins une partie de l'espace (9) avec la lumière ultraviolette (7), ou pour éclairer au moins une partie d'un espace principal (10) comprenant l'espace (9) avec la lumière ultraviolette (7).

7. Système selon l'une quelconque des revendications précédentes, dans lequel le dispositif d'éclairage (1, 21) comprend un purificateur pour purifier l'air en fonctionnement, dans lequel le purificateur comprend la source lumineuse (5), dans lequel la lumière ultraviolette (7) de la source lumineuse (5) purifie l'air.

8. Système selon l'une quelconque des revendications précédentes, dans lequel le dispositif d'éclairage (21) comprend le dispositif de commande (23).

9. Système selon l'une quelconque des revendications précédentes, dans lequel l'espace (29) comprend une surface (38), dans lequel le dispositif d'émulation (32) est configuré pour projeter ledit signal d'émulation (26) sur la surface (38).

10. Système selon l'une quelconque des revendications précédentes, dans lequel le dispositif d'émulation (2) est agencé à une distance du capteur (4), dans lequel ladite distance est d'au moins deux mètres.

11. Système selon l'une quelconque des revendications précédentes, dans lequel le système
(3000) comprend un second dispositif d'éclairage (3200) ;
dans lequel le second dispositif d'éclairage (3200) comprend le dispositif d'émulation (312).

12. Système selon l'une quelconque des revendications précédentes 1 à 9, dans lequel le dispositif d'éclairage (21) comprend le dispositif d'émulation (22).

13. Système selon l'une quelconque des revendications précédentes, dans lequel le dispositif d'éclairage (21) est un luminaire et comprend un boîtier de luminaire (34), dans lequel le boîtier de luminaire (34) comprend le capteur (24), la source lumineuse (25), le dispositif de commande (23) et le dispositif d'émulation (22).

14. Agencement de système comprenant une pluralité de systèmes (3100, 3200, 3300) selon l'une quelconque des revendications 1 à 11 précédentes, dans lequel le dispositif d'éclairage respectif d'au moins un système de la pluralité de systèmes (3100, 3200, 3300) comprend le dispositif d'émulation respectif d'un autre système de la pluralité de systèmes (3100, 3200, 3300).

15. Procédé (900) de détermination d'un état d'un capteur (4) d'un dispositif d'éclairage (1, 21) ;
dans lequel le capteur (4) est configuré pour détecter une présence dans un espace (9) et pour détecter un signal d'émulation (6),
dans lequel le procédé (900) comprend :
- la commande (901) d'un dispositif d'émulation (2) pour transmettre le signal d'émulation (6) à un premier instant ;
- la détermination (902) d'une condition de vérification du capteur (4) si le capteur (4) détecte le signal d'émulation (6) au premier instant, et la détermination (902) d'une condition d'erreur du capteur
(4)
si le capteur (4) ne détecte pas le signal d'émulation (6) au premier instant ;
- la fourniture (903) d'un signal de sortie (8) lors de la détermination de ladite condition d'erreur ;
**caractérisé en ce que,**
le dispositif d'éclairage (1, 21) comprend une source lumineuse (5) configurée pour fournir une lumière ultraviolette (7) en fonctionnement et pour interrompre la fourniture de ladite lumière ultraviolette (7) en fonctionnement si le capteur (4) détecte une présence dans ledit espace (10).
